# EUROPEAN PATENT SPECIFICATION

(11) **EP 4 353 189 B1**
(45) Date of publication and mention of the grant of the patent: **26.08.2026**
(21) Application number: 23201928.1
(22) Date of filing: 05.10.2023
(51) Int. Cl.: A61D 9/00, A61F 5/01

(54) **ORTHOSIS, IN PARTICULAR FOR ANIMALS**
ORTHESE, INSBESONDERE FÜR TIERE
ORTHÈSE, EN PARTICULIER POUR ANIMAUX

(30) Priority: 11.10.2022 PL 44249422
(43) Date of publication of application: 17.04.2024
(73) Proprietor: WIMBA POLAND spolka z ograniczona odpowiedzialnoscia, 31-553 Krakow (PL)
(72) Inventor: Kruzlak, Sylwia, 34-212 Bienkowka (PL); Zabawa, Filip, 33-112 Tarnowiec (PL)
(74) Representative: Górska, Anna

(56) References cited:
- WO-A1-2022/046043
- CN-A- 110 680 590
- KR-B1- 102 268 890
- US-A1- 2013 253 394
- US-A1- 2014 039 367
- US-A1- 2018 078 352

## Description

The object of the invention is an orthosis, in particular for animals, preferably applicable as a metacarpophalangeal joint orthosis, a radiocarpal joint orthosis, an elbow joint ortheois, a glenohumeral joint orthosis, a hip joint orthosis, a knee joint orthosis, a tarsometatarsal joint orthosis, or a tarsometatarsal joint or metatarsophalangeal joint orthosis.

From Chinese patent application no. CN110680590 there is known a knee joint orthosis for pets, and a manufacturing method thereof. By performing three-dimensional scanning and reconstruction of the upper and lower limbs of a dog's knee joint, it is possible to design and manufacture a knee joint orthosis for a dog in a manner adjusted to its needs. Based on the three-dimensional moulding technology, the upper module and the lower module of the joint can better adjust themselves to the four limbs of a dog by cooperation of fastening bandages, due to which it is possible to avoid other negative effects, such as loosening, the dog's discomfort and the like, caused by a difference in shape. The upper module and the lower module of the articulated joint are connected by means of an adjustable set of hinges, due to which the tension of the dog's knee joint can be dispersed, which allows for reducing the load applied to the dog's knee joint. Moreover, the fit between the upper connecting disc and the lower connecting disc can be adjusted so as to realise three modes of motion, namely free rotation, limited rotation and immobilisation; and in this manner, it is possible to satisfy the needs of knee joints of dogs suffering from various diseases.

From American patent application no. US2013253394 A there is in turn known an orthopaedic product for humans having longitudinal spacers, each of which has a tape-coupling element, and a flexible tape mounting the spacers by means of the tape-coupling elements. A semi-rigid and flexibly bent expander is connected to the spacers, and it extends over a specified length therebetween. The expander provides two main positions of the orthopaedic product: stiffening in a straight and bent position of the limb.

On the other hand, from American patent application no. US2014039367 A there is known an orthopaedic device for use on an animal's limb, which provides stability of unstable joints and limbs for short- or long-term support and functioning. This device is designed so as to prevent the occurrence of or reduce the intensity of a joint injury, stabilising the joint, at the same time assisting in or restricting the range of movement. The device can comprise an adjustable tensioning system, which is meant to provide an individual fit for a wide range of animals and injuries.

From application no. WO22046043 A there is also known a knee joint orthosis with adjustable tension, used to relieve a knee joint affected by arthrosis, thus reducing the pain and improving mobility, which consists of: an upper and lower frame connected by a set of relieving hinges, and a set of hinges with adjustable tension, optionally comprising a sensor and enabling remote or automatic tension control of the orthosis.

The technical problem is the development of a new orthosis with a simple design and economical to produce, which enables both immobilisation of a limb in a joint, as well as its partial or complete bending, according to the medical needs of the patient.

The essence of the orthosis according to the invention is that it comprises a frame comprising straps with fasteners, hinges and lateral elements connecting the straps to the hinges, each of which consists of a sleeve with an arcuate shape connected to one lateral element, and a hoop connected to the other lateral element, the hoop being placed in the sleeve with a clearance fit; moreover, the orthosis is provided with removable stops that are placed in hinges with various degrees of fits that are provided with grooves, and a rim provided with recesses, with a width corresponding to at least the width of the lateral elements with fit tolerance, as well as a protrusion situated in the central part of the stop.

Preferably, the fastener of the strap consists of an opening on one end of the strap, and a protrusion on the other end thereof.

Preferably, the straps are provided with mounting openings.

Preferably, the straps are provided with pads on the inner side.

Preferably, the stops have the shape of discs.

Preferably, the frame is manufactured entirely as a single flat-printed element.

The orthosis according to the invention is presented in an embodiment in the attached drawing, in which:
- Fig. 1: presents an unfolded frame **1** of the orthosis;
- Fig. 2: presents a fastened orthosis in a side view, provided with stops **3** and pads **2;**
- Fig. 3: presents a fastened orthosis provided with stops **3** and pads **2** in an isometric view;
- Fig. 4: presents a fastened orthosis provided with stops **3** and pads **2** in a rear view;
- Fig. 5: presents a fastened orthosis provided with pads **2** in another embodiment, put on a limb of a dog, without stops **3;**
- Fig. 6: presents a stop **3** blocking the bending;

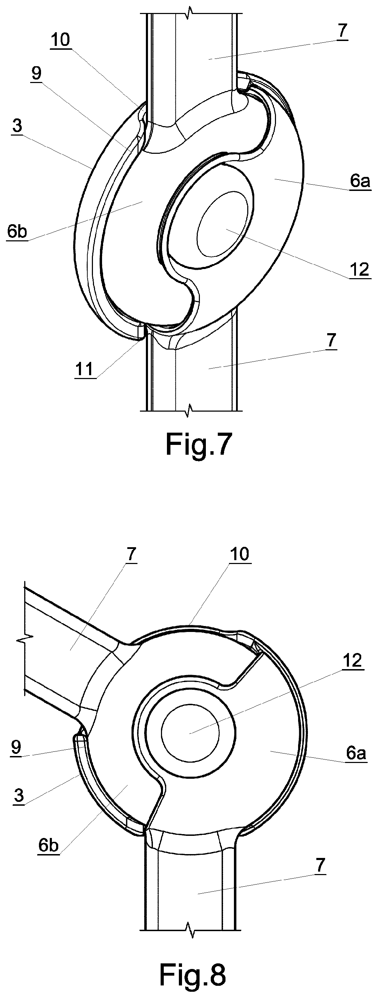

- Fig. 7: presents a stop **3** blocking the bending, placed in a hinge **6;**
- Fig. 8: presents a stop **3** restricting the range of bending, placed in a hinge **6;**

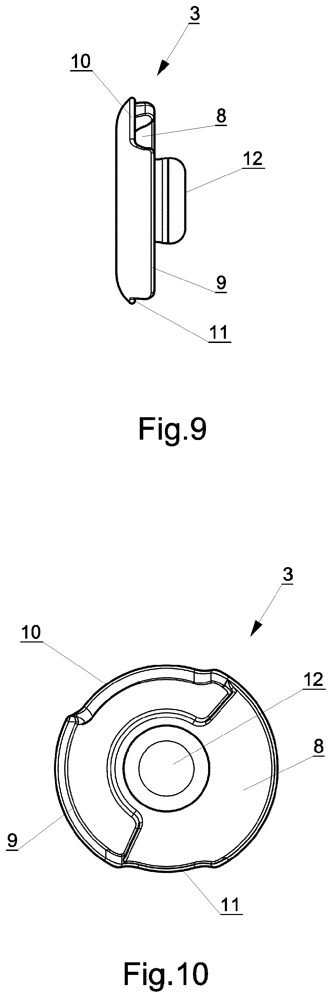

- Fig. 9: presents a stop **3** restricting the range of bending in a side view;
- Fig. 10: presents a stop **3** restricting the range of bending in a front view.

The orthosis according to the invention and as presented in the attached drawing comprises a frame **1,** pads **2** and stops **3.** The frame **1** comprises straps **4** with fasteners (consisting of an opening **5a** on one end of the strap **4** and a protrusion **5b** on the other end thereof), hinges **6** and lateral elements **7** connecting the straps **4** to the hinges **6.** The frame **1** is preferably made using the Multi Jet Fusion (MJF) printing technology from the PA11 material, as a flat element with flexibility enabling the fastening of the straps **4,** i.e. bending them so that it would be possible to place the protrusions **5a** in the openings **5b.** The straps are further provided with mounting openings **13, 14** used to mount the pads **2.** Such form of the frame **1** enables using the orthosis according to its purpose, since in this form the axes of the hinges **6** are arranged parallel to each other and perpendicularly to the axis of the limb placed in the orthosis, and can therefore be bent. Each of the hinges **6** consists of a sleeve **6a** with an arcuate shape connected to one lateral element **7,** and a hoop **6b** connected to the other lateral element **7,** the hoop **6b** being placed in the sleeve **6a.** The entire frame **1,** i.e. the straps **4,** the fasteners, the hinges **6** and the lateral elements **7,** may be manufactured as a single flat-printed element, and does not require additional connection or assembly of the individual components.

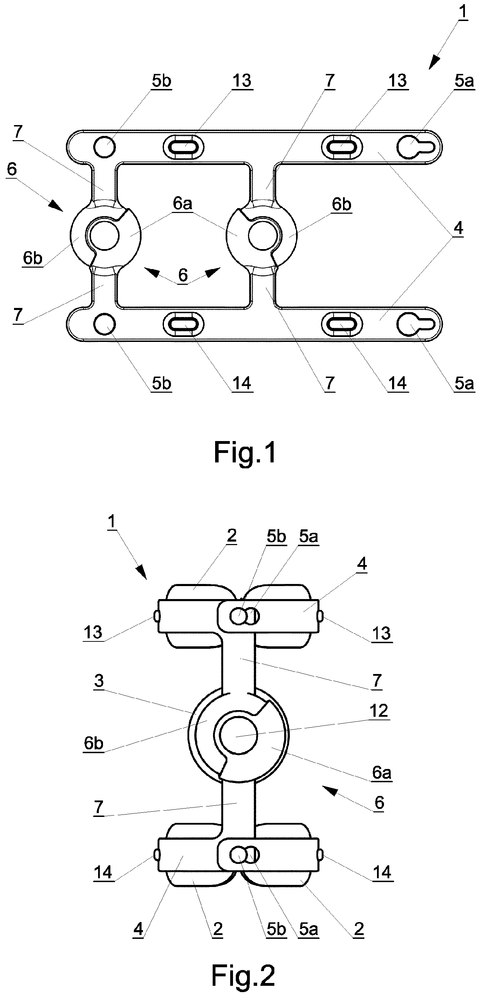

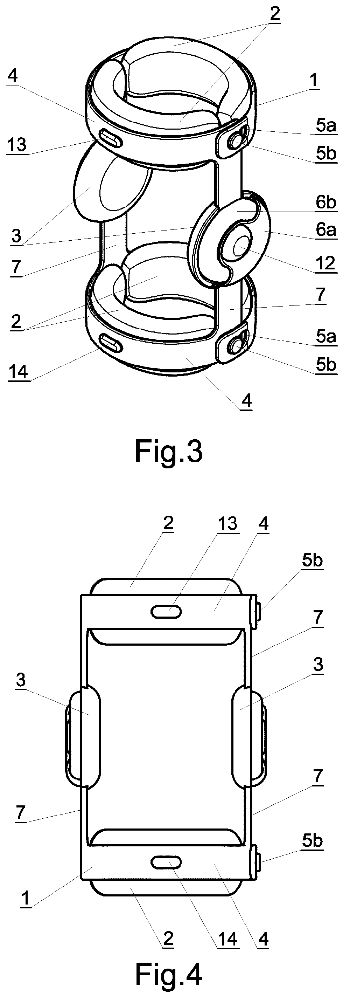

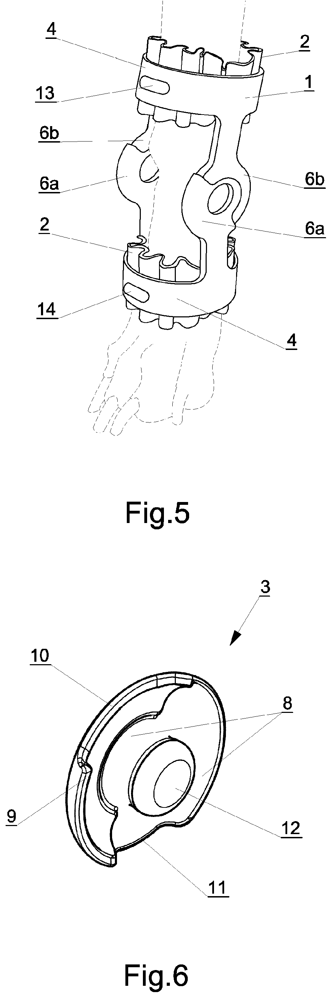

The flexible material from which the frame **1** is made allows for putting the orthosis on and fastening it on a limb. The orthosis is put on an animal's limb so that the hinges **6** are positioned next to the animal's joint, and the straps **4** surround the limb on both sides of this joint. The inner surface of the straps **4** is provided with a pad **2,** preferably soft, printed from the TPU material. The pad can have a shape similar to a cushion, as indicated in Figs. 2-4, or an accordion, as presented in Fig. 5. The pad **2** allows for putting the orthosis stably on an animal's limb, increases the comfort of usage due to the use of a soft material, and enables a precise fit of the orthosis on the limb. The stops **3** preferably have the form of discs provided with grooves **8** on at least one of their surfaces, and their edges are provided with a rim **9** provided with recesses **10, 11** with a width corresponding to at least the width of the lateral elements **7** with fit tolerance. Depending on the degree of exposing the edge of the hinge **6,** the lengths of the recesses **10, 11** in the rim **9** comprising the hinge **6** affect the range of its movement, meaning the longer the recesses **10, 11,** the wider the range of movement of the hinge **6.** In the central part of the stop **3** there is a protrusion **12** allowing for immobilisation of the stop **3** in the hinge **6.** When put on, the protrusion **12** passes through the centre of the hinge axis, preferably deeper than half of its cross-section. The grooves **8** allow for placing the stop **3** in the hinge **6;** the recesses **10, 11** in the rim **9** prevent bending, or restrict its range. By various degrees of fits (from clearance to interference), the stops **3** can also increase the resistance accompanying the bending of the hinge **6,** which can be desired when healing certain dysfunctions.

The stops **3** are preferably made using the MJF printing technology from PA12 (colour printing), PA11 (high stiffness and durability) and TPU materials (using the friction and flexibility of the material). Due to the various developed designs of the stops **3,** in particular due to the ability to use various lengths and placements of the recesses **10, 11,** it is possible to use the orthosis for various purposes, both for completely stiffening the joint, with simultaneous control of its bending angle, as well as for dysfunction therapy, by controlling the range of an animal's movement, as well as controlling the resistances which the orthosis presents to the animal, which is achieved through various degrees of fits of the stops 3 (from clearance to interference).

Injuries of the lower limbs of small animals often require therapy using an orthosis or stiffening. The orthosis according to the invention can be used on the lower part of a front or hind leg, in order to provide its stability and support. The orthoses can be available in a full range of sizes adjusted to a specific animal, in order to provide comfort and effectiveness of use. The orthoses according to the invention can be found useful in helping to cure wrist hyperextension, radiocarpal joint instability, arthrosis, a damaged lateral wrist ligament, as well as for postoperative support of wrist arthrodesis or its failure, and can also be used in prophylaxis as support during amputation of a contralateral limb, during foot injuries, including a damaged tendon and amputation of the toes, during peripheral neuropathy and distal neuropathy of the brachial plexus. The frame **1** of the orthosis has a wide range of uses, depending on the type of the used stops **3.** The orthosis can be made in particular in the 3D printing technology, in which the frame **1** is made of a single component in the same technological process. Similarly, the pads **2** and the stops **3** are made of a single material. The orthosis according to the invention is preferably manufactured on the basis of a limb scan, with no need to make physical casts or shells, e.g. made from plaster. The frame 1 of the orthosis according to the invention may be printed as a flat element, which when bent in order to be put on a limb allows for movement in the hinges **6.** The orthosis according to the invention can be made in a plurality of sizes, including individually, according to the dimensions of a specific animal. Adjustment of the orthosis to the dimensions of a specific animal is achieved by adjusting the length of the straps **4** or the lateral elements **7.** According to international tolerance classes which are established in ISO 286, the scope of variance of the dimensions of individual elements of the orthosis corresponds to class IT 13.

## Claims

1. Orthosis comprising a frame comprising straps with fasteners and hinges, **characterised in that** the frame (1) comprises straps (4) with fasteners, hinges (6) and lateral elements (7) connecting the straps (4) to the hinges (6), each of which consists of a sleeve (6a) with an arcuate shape connected to one lateral element (7), and a hoop (6b) connected to the other lateral element (7), the hoop (6b) being placed in the sleeve (6a) with a clearance fit; moreover, the orthosis is provided with removable stops (3) that are placed in hinges (6) with various degrees of fits that are provided with grooves (8), and a rim (9) provided with recesses (10, 11), with a width corresponding to at least the width of the lateral elements (7) with fit tolerance, and a protrusion (12) situated in the central part of the stop (3).

2. The orthosis according to claim 1, **characterised in that** the fastener of the strap (4) consists of an opening (5a) on one end of the strap (4), and a protrusion (5b) on the other end thereof.

3. The orthosis according to claim 1, **characterised in that** the straps (4) are provided with mounting openings (13, 14).

4. The orthosis according to claim 1, **characterised in that** the straps (4) are provided with pads (2) on the inner side.

5. The orthosis according to claim 1, **characterised in that** the stops (3) have the shape of discs.

6. The orthosis according to claim 1, **characterised in that** the frame (1) is manufactured entirely as a single flat-printed element.

## Patentansprüche

1. Orthese, bestehend aus einem Rahmen mit Riemen, Verschlüssen und Scharnieren, **dadurch gekennzeichnet, dass** der Rahmen (1) Riemen (4) mit Verschlüssen, Scharniere (6) und seitliche Elemente (7) umfasst, die die Riemen (4) mit den Scharnieren (6) verbinden, von denen jedes aus einer mit einem seitlichen Element (7) verbundenen, bogenförmigen Hülse (6a) und einem mit dem anderen seitlichen Element (7) verbundenen Bügel (6b) besteht, wobei der Bügel (6b) mit Spiel in die Hülse (6a) eingesetzt ist; die Orthese ferner mit abnehmbaren Anschlägen (3) versehen ist, die in Scharnieren (6) mit unterschiedlichen Passungsgraden angeordnet sind, die mit Nuten (8) versehen sind, sowie mit einem Rand (9), der mit Aussparungen (10, 11) versehen ist, deren Breite mindestens der Breite der seitlichen Elemente (7) mit Passungstoleranz entspricht, und mit einem Vorsprung (12), der sich im mittleren Teil des Anschlags (3) befindet.

2. Orthese nach Anspruch 1, **dadurch gekennzeichnet, dass** der Verschluss des Riemens (4) aus einer Öffnung (5a) an einem Ende des Riemens (4) und einem Vorsprung (5b) an dessen anderem Ende besteht.

3. Orthese nach Anspruch 1, **dadurch gekennzeichnet, dass** die Riemen (4) mit Befestigungsöffnungen (13, 14) versehen sind.

4. Orthese nach Anspruch 1, **dadurch gekennzeichnet, dass** die Riemen (4) an der Innenseite mit Polstern (2) versehen sind.

5. Orthese nach Anspruch 1, **dadurch gekennzeichnet, dass** die Anschläge (3) die Form von Scheiben haben.

6. Orthese nach Anspruch 1, **dadurch gekennzeichnet, dass** der Rahmen (1) vollständig als ein einziges flachgedrucktes Element hergestellt ist.

## Revendications

1. Une orthèse comprenant un cadre comprenant des sangles avec des attaches et des charnières, **caractérisée en ce que** le cadre (1) comprend des sangles (4) avec des attaches, des charnières (6) et des éléments latéraux (7) reliant les sangles (4) aux charnières (6), chacune d'entre elles étant constituée d'un manchon (6a) de forme arquée relié à un élément latéral (7), et d'un anneau (6b) relié à l'autre élément latéral (7), l'anneau (6b) étant placé dans le manchon (6a) avec un ajustement lâche ; de plus, l'orthèse est munie de butées amovibles (3) qui sont placées dans des charnières (6) présentant différents degrés d'ajustement et pourvues de rainures (8), ainsi que d'un rebord (9) muni d'évidements (10, 11) dont la largeur correspond au moins à la largeur des éléments latéraux (7) avec une tolérance d'ajustement, et d'une saillie (12) située dans la partie centrale de la butée (3).

2. L'orthèse selon la revendication 1, **caractérisée en ce que** l'attache de la sangle (4) comprend une ouverture (5a) à une extrémité de la sangle (4) et une saillie (5b) à l'autre extrémité de celle-ci.

3. L'orthèse selon la revendication 1, **caractérisée en ce que** les sangles (4) sont pourvues d'ouvertures de montage (13, 14).

4. L'orthèse selon la revendication 1, **caractérisée en ce que** les sangles (4) sont pourvues de coussinets (2) sur leur face intérieure.

5. L'orthèse selon la revendication 1, **caractérisée en ce que** les butées (3) ont la forme de disques.

6. L'orthèse selon la revendication 1, **caractérisée en ce que** le cadre (1) est entièrement fabriqué comme un seul élément imprimé à plat.
